# EUROPEAN PATENT SPECIFICATION

(11) **EP 3 431 468 B1**
(45) Date of publication and mention of the grant of the patent: **09.06.2021**
(21) Application number: 17305953.6
(22) Date of filing: 18.07.2017
(51) Int. Cl.: C07D 211/58, C07D 401/12, A61K 31/4468, A61P 25/24, A61P 25/28

(54) **USE OF ADIPONECTIN RECEPTOR AGONISTS IN TREATING DEPRESSION, ANXIETY AND NEUROINFLAMMATION**
VERWENDUNG VON ADIPONECTINREZEPTORAGONISTEN ZUR BEHANDLUNG VON DEPRESSIONEN, ANGSTZUSTÄNDEN UND NERVENENTZÜNDUNGEN
UTILISATION D'AGONISTES DU RÉCEPTEUR DE L'ADIPONECTINE POUR LE TRAITEMENT DE LA DÉPRESSION, L'ANXIÉTÉ ET LA NEURO-INFLAMMATION

(43) Date of publication of application: 23.01.2019
(73) Proprietor: CENTRE NATIONAL DE LA RECHERCHE SCIENTIFIQUE, 75016 Paris (FR); Université Nice Sophia Antipolis, 06103 Nice Cedex 2 (FR)
(72) Inventor: NICOLAS, Sarah, 06250 Mougins (FR); GUYON, Alice, 06600 Antibes (FR); CHABRY, Joëlle, 06140 Tourrettes sur Loup (FR)
(74) Representative: Lavoix

(56) References cited:
- EP-A1- 3 053 911
- FILIPPO WEISZ ET AL: "The role of adiponectin receptors in the regulation of synaptic transmission in the hippocampus : WEISZ et al.", SYNAPSE, vol. 71, no. 5, 6 February 2017 (2017-02-06), page e21964, XP055438620, US ISSN: 0887-4476, DOI: 10.1002/syn.21964
- D ZHANG ET AL: "Adiponectin regulates contextual fear extinction and intrinsic excitability of dentate gyrus granule neurons through AdipoR2 receptors", MOLECULAR PSYCHIATRY, vol. 22, no. 7, 1 July 2017 (2017-07-01), pages 1044-1055, XP055438624, GB ISSN: 1359-4184, DOI: 10.1038/mp.2016.58
- YOSHIKI TAKAMATSU ET AL: "Protection against neurodegenerative disease on Earth and in space", NPJ MICROGRAVITY, vol. 2, no. 1, 7 April 2016 (2016-04-07), XP055438622, DOI: 10.1038/npjmgrav.2016.13

## Description

The invention provides adiponectin receptor agonists, for use in treating depression, anxiety or neuroinflammation.

### BACKGROUND OF THE INVENTION

Depression (major depressive disorder, MDD) is a mood disorder of multifactorial origin, including genetic and environmental factors, estimated to affect 350 million people worldwide (Chabry et al., 2015). Major depression is responsible for increased morbidity and mortality, adverse health behaviors, lost work productivity and social and familial unrests (Benton et al., 2007).

Current treatments mainly based on monoaminergic system regulation such as monoamine oxidase inhibitors (MAOIs) and selective serotonin reuptake inhibitors (SSRIs) are available; however, about 30% of patients with depressive disorders are partially or completely resistant to treatments. Thus, identification of novel therapeutic targets for the treatment of these disorders is urgently needed.

Beside monoaminergic system dysregulation, depression has been linked to dysfunction of the hypothalamo-pituitary-adrenal (HPA) axis and structural changes within the hippocampus mainly linked to a neurogenesis weakening. Patients with chronic inflammation are often associated with the emergence of depression symptoms, while diagnosed depressed patients show increased plasma levels of pro-inflammatory cytokines. Moreover, activation of microglial cells and astrocytes as well as increased levels of pro-inflammatory cytokines detected *post mortem* in brain of depressed patients strongly suggest a crucial role for neuroinflammation in depression pathogenesis.

Weisz et al, Synapse 2017, 71(5), e21964(18), Zhang et al Molec. Psychiatry, 2017, 22(7), 1044-55, and Takamatsu et al, Microgravity, 2016, 2(1), 16013 (1-5) disclose AdipoR agonists such as AdipoRon and suggest its use in therapeutic strategies.

### SUMMARY OF THE INVENTION

The invention provides adiponectin receptor agonists for use in treating depression, anxiety or any neuroinflammatory disorders, such as neurodegenerative disorders with a neuroinflammatory component, wherein the adiponectin receptor agonist is a compound of formula (1): 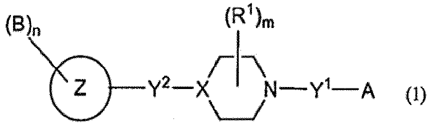 Wherein
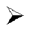 A represents a phenyl group optionally substituted with an halogen atom or a methyl group,
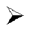 Y¹ represents - (CHR²)ₐ -, wherein R² represents H or a methyl group and a represents 1;
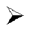 X is CH;
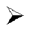 R¹ represents a C₁₋₇ alkyl group with m representing 0;
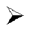 Y² represents *-O-CH₂-CONH;;
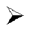 Z represents a phenyl group
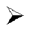 (B)ₙ represents -CO-R⁴ or -O-R⁴, wherein R⁴ is chosen from:
   - a phenyl group optionally substituted with a halogen atom, or
   - a pyridyl group optionally substituted with a halogen atom, and ;
      n represents 1.

It is disclosed compounds of formula (I) above where
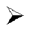 A represents a substituted or unsubstituted aryl group, a substituted or unsubstituted heteroaryl group, a substituted or unsubstituted aryloxy group, or a C₄₋₈ tertiary alkyl group or -NH₂ ;
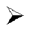 Y¹ represents - (CHR²)ₐ -, wherein R² represents H or a C₁₋₇ alkyl group, and a represents an integer of 0 to 2, or -CO-;
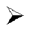 X represents CH or N;
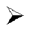 R¹ represents a C₁₋₇ alkyl group, and a plurality of R¹s may be the same or different from
each other and m represents an integer of 0 to 4
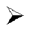 Y² represents:
   - if X is CH,
      then *-O-CH₂-CONH-, -O-, *-CONH-, or 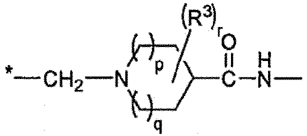 wherein R³ represents a C₁₋₇ alkyl group, r represents an integer of 0 to 4, and p and q independently represents an integer of 0 to 2, in which if r is 2 or more, R³s may be the same or different from each other, and * represents a site bonding to Z;
   - if X is N,
      then *-CONH-(CH₂)_{b}-CO-, *-NHCO-Ar¹-CH₂-, *-NHCO-(CH₂)_{b}-, or -CO-, wherein Ar¹ represents a substituted or unsubstituted arylene group, b represents an integer of 1 to 3, and * represents a site bonding to Z;
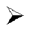 Z represents a cyclic group selected from the group consisting of an aryl group, a heteroaryl group and a C₃₋₇ cycloalkyl group;
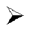 B may be a substituent of the cyclic group Z, and represents -CO-R⁴; -O-R⁴, wherein R⁴ represents a C₁₋₇ alkyl group, a substituted or unsubstituted phenyl group, or a substituted or unsubstituted pyridyl group; -CONR⁵R⁶, wherein R⁵ represents H, a C₃₋₇ cycloalkyl group, a C₁₋₄ alkoxy C₁₋₄ alkyl group, a norbornyl C₁₋₄ alkyl group, or a Ar²-C₁₋₄ alkyl group in which Ar² is a substituted or unsubstituted aryl group or a heteroaryl group, and R⁶ represents H, a C₁₋₇ alkyl group, a C₂₋₄ alkenyl group or a C₂₋₄ alkynyl group; a C₁₋₇ alkyl group; a C₃₋₇ cycloalkyl group; a halo C₁₋₇ alkyl group; phenyl group; a halogen atom; -NO₂; or a group shown as follows; and 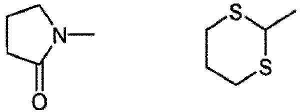
n represents an integer of 0 to 3, in which if n is 2 or more, Bs may be the same or different from each other;
or a pharmaceutically acceptable salt thereof.

In a most preferred embodiment, the adiponectin receptor agonist is 2-(4-Benzoylphenoxy)-N-[1-(phenylmethyl)-4-piperidinyl]-acetamide (also named AdipoRon).

### LEGENDS TO THE FIGURES

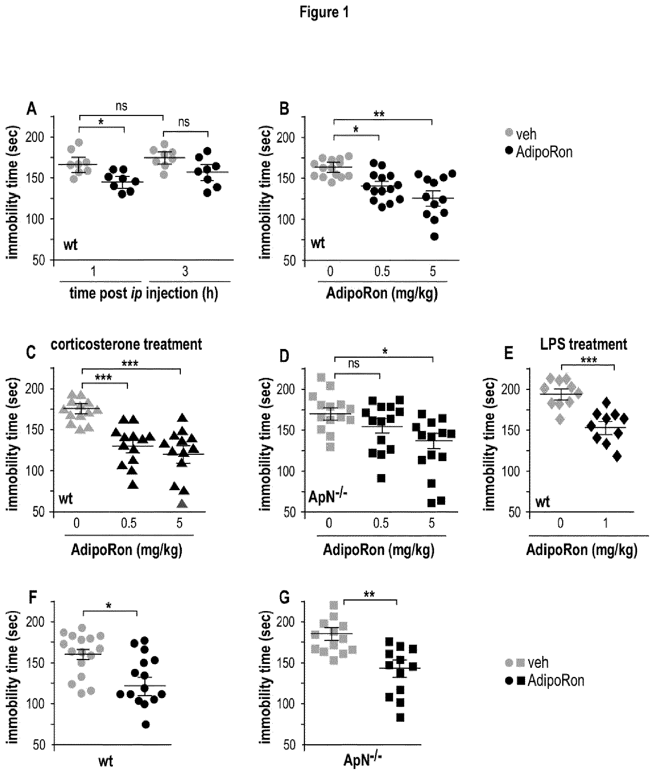

**Figure 1****: Assessment of the antidepressant-like effect of an acute injection of AdipoRon using the FST paradigm.** Ten weeks old male wt mice were *ip* injected with Adiporon (1mg/kg in 2.5% DMSO, dark gray) or vehicle alone (light gray) one and three hours prior the FST performed as described in Example 1 (**A**). Male wt mice untreated (**B**) or long-term treated with corticosterone in the drinking water (**C**) and ApN^{-/-} (**D**) male mice were *ip* injected with the indicated concentration of AdipoRon (0.5 and 5mg/kg in 2.5% DMSO) or vehicle alone (0) one-hour prior the FST. (**E**) Wt male mice were *ip* injected with LPS (0.83 mg/kg), sixteen-hours later, mice were *ip* injected with AdipoRon (1mg/kg) or vehicle alone prior to be submitted to the FST. AdipoRon (1mg/kg) or vehicle alone was administrated by gavage to ten weeks old male wt (**F**) and ApN^{-/-} mice (**G**) one-hour prior the FST. The immobility time (in sec) during the last four minutes out of six of the test was timed. Data are plotted as mean ± SEM; each symbol represents a mouse; Kruskal-Wallis followed by a Dunn's multiple comparison test. ns, non-significant ; **P* < 0.05, ***P* < 0.01 ; ****P* < 0.001.

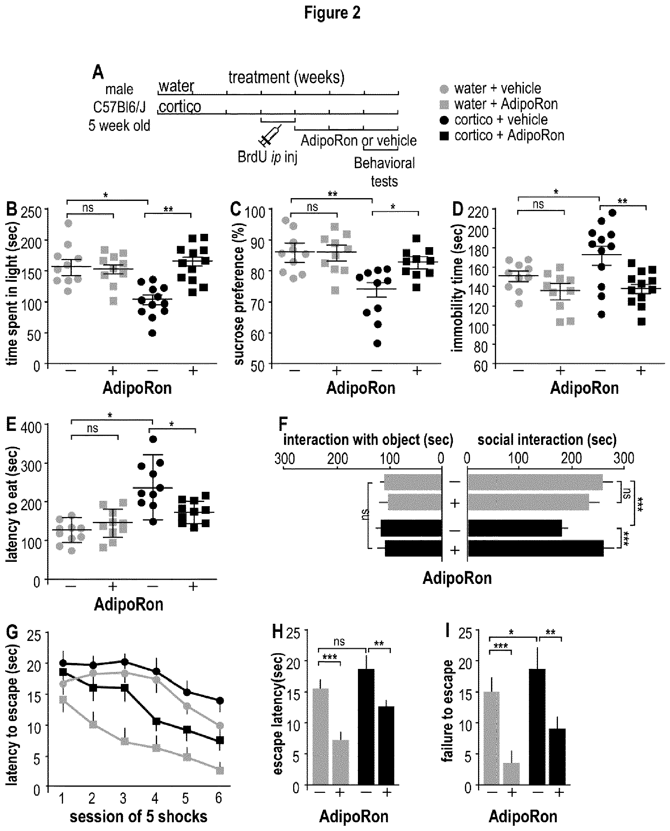

**Figure 2****: Antidepressant effects of AdipoRon assessed through behavioral tests on corticosterone-induced depressive-like mice. (A)** Schematic representation of the experimental protocol as described in the Example 1. Five week old mice were randomly distributed to the following groups i.e. untreated (light gray circle), long-term corticosterone-treated (square gray circle), daily *ip* injected with AdipoRon (1mg/kg, dark gray circle) or vehicle alone (dark gray square). Independent groups of mice were submitted to the following experiments i- BrdU was *ip* administrated to mice for five consecutive days twenty days prior euthanasia then brains were collected and sliced into 30µm sections. Slices were proceed to immunohistochemical detection of BrdU⁺ positive cells within the dente gyrus ii- during the seventh week of treatment, behavioral testing were performed including successively the open field, the black and white test, the rotarod, the social interaction test, the NSF test and the FST iii- an independent group of animal was submitted the sucrose preference test and the learned helplessness test for five consecutive days. Effect of chronic treatment with AdipoRon (1mg/kg) was assessed on untreated (-) or long-term corticosterone treated (+) mice through the following behavioral tests; in the light and dark **(B),** sucrose preference **(C),** FST **(D),** NSF **(E),** social interactions **(F)** and learned helplessness test from which are extracted data on the latency **(G, H)** and the number of failure to escape the aversive box **(I).** Values plotted are mean ± SEM; each symbol represents a mouse (N=10-12 *per* group); Kruskal-Wallis statistical analysis was done followed by a Dunn's statistical test for comparison between groups, ns, non-significant; **P* < 0.05, ***P* < 0.01 ; ****P* < 0.001.

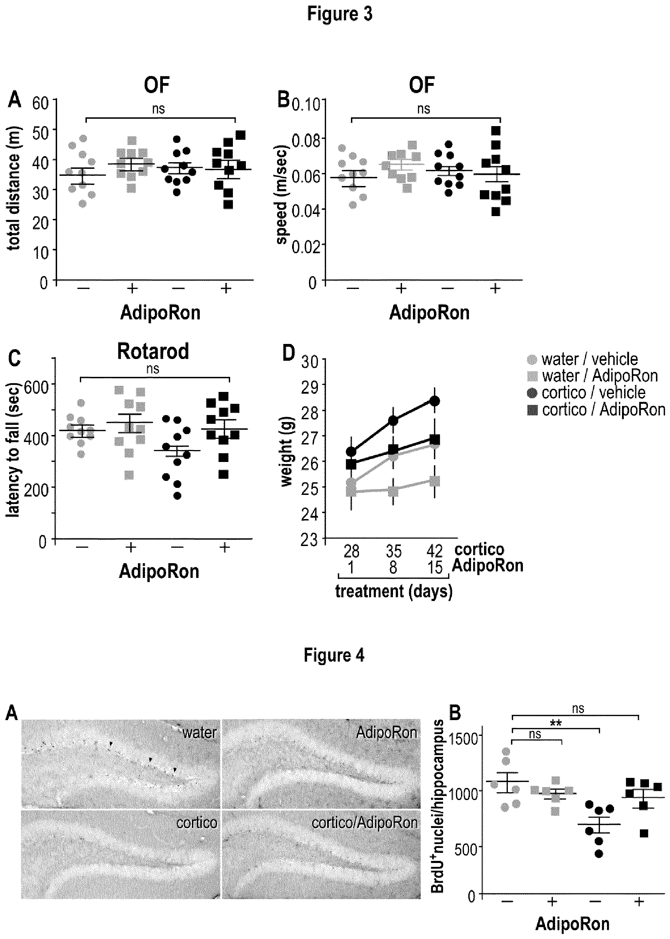

**Figure 3****: Lack of effect of AdipoRon on locomotor and motor coordination.** Locomotor and exploratory activities (**A, B**) and motor coordination (**C**) of wt mice chronically treated with vehicle or AdipoRon (*ip,* 1mg/kg, for twenty consecutive days) were recorded in the open-field (OF) for 10 minutes (i.e. total distance **(A)** and mean speed (**B**) and in the rotarod (**C**) respectively.
**Figure 4****: AdipoRon rescues the neurogenesis in the dentate gyrus of the hippocampus of corticosterone-treated mice. A.** Representative photomicrographs of BrdU-labeled nuclei (*arrowheads*) of dentate gyrus slices from brains of untreated (*up*) or chronically corticostreone-treated (*down*) mice treated with AdipoRon (*left*) or vehicle (*right*). **B.** Data represent as the number of BrdU-positive nuclei counted in the dentate gyrus of the four groups of mice. Values plotted are mean ± SEM; ns, non-significant; ***P*< 0.01; N = 6 mice in each group; each symbol represents a mouse.

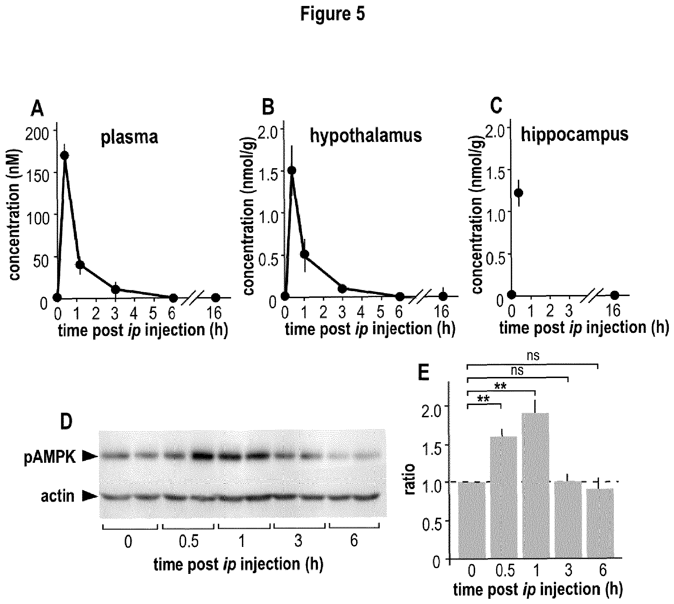

**Figure 5****: AdipoRon crosses the BBB and targets the brain.** The concentrations of AdipoRon was measured in plasma (**A**), hypothalamus (**B**) and hippocampus (**C**) by HPLC-coupled to MS-MS analysis at different time points post *ip* injection. Data are mean ± SEM area expressed in nM for the plasma and in nmol/mg of total protein for brain areas; N = 6 mice *per* group. **D.**
Representative western blot showing the kinetic of phosphorylation of AMPK in the hypothalamus from vehicle- (0) or AdipoRon-treated mice 30min (0.5), 1h, 3h or 6h post *ip* injection assessed by western blotting. **E.** Histogram showing the mean ± SEM of the four independent experiments, N=2 *per* time point *per* experiment. Kruskal-Wallis followed by a Dunn's post-hoc test for comparison with the vehicle control group; ns, non-significant; ***P <* 0.01.

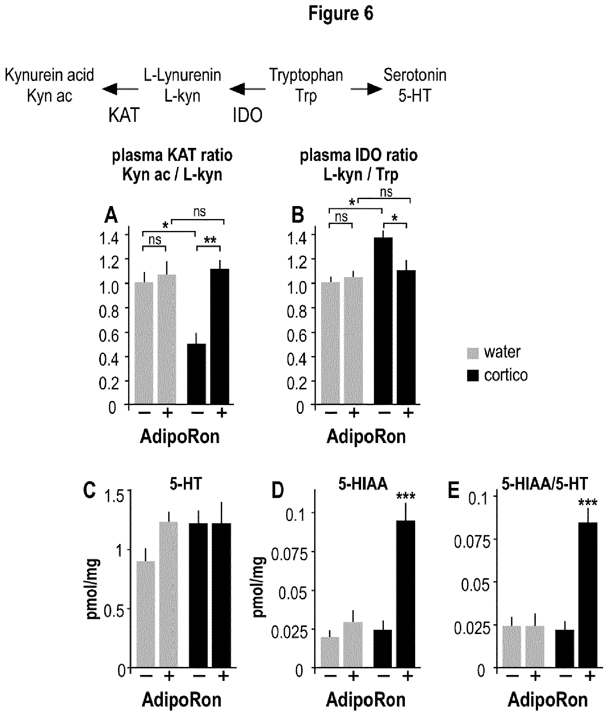

**Figure 6****: AdipoRon restores peripheral Kyn and brain 5-HT pathways altered in depression-like conditions.** Schematic representation of the Kyn pathway. Histograms representing the relative plasma ratio Kyn ac/L-Kyn (**A**) and L-Kyn/Trp (**B**) from control (*light gray*) and depressive-like mice (*dark gray*). Data from untreated control mice were taken as 1, data were expressed as mean ± SEM; N = 7 mice *per* group. Concentration (pmol/mg of total proteins) of 5-HT and its metabolite 5-HIAA in dorsal raphe from control and depressive-like mice were plotted as mean ± SEM; N = 7 mice *per* group. Kruskal-Wallis followed by a Dunn's post-hoc test for comparison with the vehicle control group; ns, non-significant; **P* < 0.05, ***P <* 0.01, ****P* < 0.001.

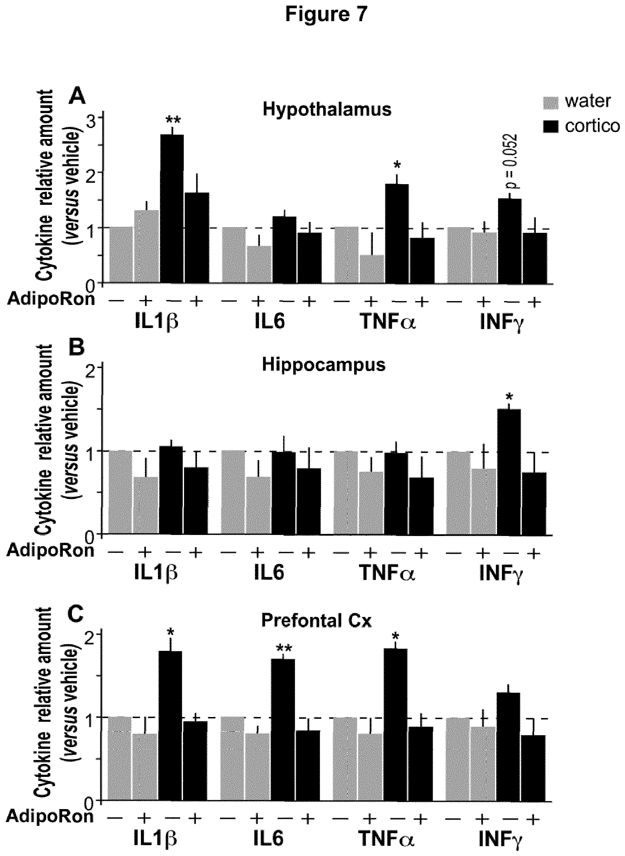

**Figure 7****: AdipoRon alleviates depression-related neuroinflammation.** Proinflammatory cytokines IL1β, IL6, TNFα, and INFγ were measured in hypothalamus (**A**), hippocampus (**B**), and prefrontal cortex (**C**) using a multiarray assay as described in the Example 1. Data expressed as relative amount (corresponding brain area from control untreated mice taken as 1) were expressed as mean ± SEM; N = 8 mice *per* group.

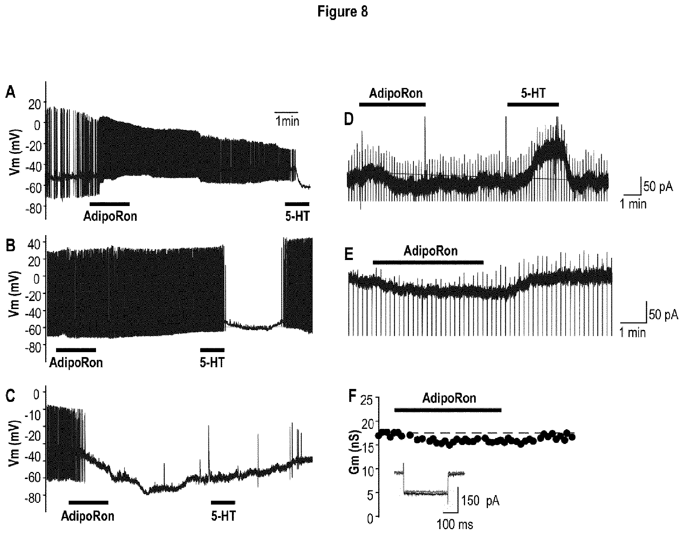

**Figure 8****: Adiporon modulates presumed serotoninergic neuron activity. A.** AdipoRon increases the frequency of discharge in action potentials and depolarizes a presumed serotoninergic neuron, characterized by its low frequency regular pattern of discharge and its response to an application of 5-HT at the end of the recording. **B.** AdipoRon has no effect on the action potential discharge in a presumed serotoninergic neuron. **C.** AdipoRon hyperpolarizes a presumed non 5-HT neuron, characterized by its lack of response to an application of 5-HT at the end of the recording. **D.** AdipoRon induces an inward current in a presumed serotoninergic neuron, characterized by an outward current induced in response to an application of 5-HT at the end of the recording. Vertical bars are rapid currents in response to hyperpolarizing pulses to measure the variations in membrane conductance of the cell. **E.** The inward current induced by AdipoRon (*top*) is accompanied by a decrease in membrane conductance (*down*) evaluated by measuring the amplitude of the current induced by a hyperpolarizing pulse of 200 ms every 10 seconds, as shown in the insert (*grey*: 10 µM AdipoRon, *black*: control DMSO).

### DETAILED DESCRIPTION OF THE INVENTION

### Definitions

In the context of the present invention, the terms below have the following meaning.

The terms mentioned herein with prefixes such as for example C₁₋₃, C₁₋₈ or C₂₋₈ can also be used with lower numbers of carbon atoms such as C₁₋₂, C₁₋₆, or C₃₋₇. If, for example, the term C₁₋₃ is used, it means that the corresponding hydrocarbon chain may comprise from 1 to 3 carbon atoms, especially 1, 2 or 3 carbon atoms. If, for example, the term C₁₋₇ is used, it means that the corresponding hydrocarbon chain may comprise from 1 to 6 carbon atoms, especially 1, 2, 3, 4, 5, 6, 7 or 8 carbon atoms. If, for example, the term C₂₋₆ is used, it means that the corresponding hydrocarbon chain may comprise from 2 to 6 carbon atoms, especially 2, 3, 4, 5 or 6 carbon atoms.

The term **"alkyl"** refers to a saturated, linear or branched aliphatic group. The term "C₁₋₃ alkyl" more specifically means methyl, ethyl, propyl, or isopropyl. The term "C₁₋₆ alkyl" more specifically means methyl, ethyl, propyl, isopropyl, butyl, isobutyl, tert-butyl, pentyl or hexyl. In a preferred embodiment, the "alkyl" is a methyl, an ethyl, a propyl, or an isopropyl, more preferably a methyl.

The term **"alkenyl"** refers to an unsaturated, linear or branched aliphatic group comprising at least one carbon-carbon double bound. The term "C₂₋₆ alkenyl more specifically means ethenyl, propenyl, isopropenyl, butenyl, isobutenyl, pentenyl, or hexenyl.

The term **"alkynyl"** refers to an unsaturated, linear or branched aliphatic group comprising at least one carbon-carbon triple bound. The term "C₂₋₆ alkynyl more specifically means ethynyl, propynyl, butynyl, pentynyl, isopentynyl, or hexynyl.

The term **"alkoxy"** or **"alkyloxy"** corresponds to the alkyl group as above defined bonded to the molecule by an -O- (ether) bond. C₁₋₃ alkoxy includes methoxy, ethoxy, propyloxy, and isopropyloxy. C₁₋₆ alkoxy includes methoxy, ethoxy, propyloxy, isopropyloxy, butyloxy, isobutyloxy, tert-butyloxy, pentyloxy and hexyloxy. In a preferred embodiment, the "alkoxy" or "alkyloxy" is a methoxy.

The term **"cycloalkyl"** corresponds to a saturated or unsaturated mono-, bi- or tri-cyclic alkyl group comprising between 3 and 20 atoms of carbons. It also includes fused, bridged, or spiro-connected cycloalkyl groups. The term "cycloalkyl" includes for instance cyclopropyl, cyclobutyl, cyclopentyl, and cyclohexyl. The term "cycloalkyl" may also refer to a 5-10 membered bridged carbocyclyl such as bicyclo[2,2,1]heptanyl, bicyclo[2,2,2]octanyl, or adamantyl, preferably bicyclo[2,2,2]octanyl. In a preferred embodiment, the "cycloalkyl" is a cyclopentyl or a cyclohexyl.

The term **"heterocycloalkyl"** corresponds to a saturated or unsaturated cycloalkyl group as above defined further comprising at least one heteroatom such as nitrogen, oxygen, or sulphur atom. It also includes fused, bridged, or spiro-connected heterocycloalkyl groups. Representative heterocycloalkyl groups include, but are not limited to 3-dioxolane, benzo [1,3] dioxolyl, pyrazolinyl, pyranyl, thiomorpholinyl, pyrazolidinyl, piperidyl, piperazinyl, 1,4-dioxanyl, imidazolinyl, pyrrolinyl, pyrrolidinyl, piperidinyl, imidazolidinyl, morpholinyl, 1,4-dithianyl, pyrrolidinyl, oxozolinyl, oxazolidinyl, isoxazolinyl, isoxazolidinyl, thiazolinyl, thiazolidinyl, isothiazolinyl, isothiazolidinyl, dihydropyranyl, tetrahydro-2H-pyranyl, tetrahydrofuranyl, and tetrahydrothiophenyl. The term "heterocycloalkyl" may also refer to a 5-10 membered bridged heterocyclyl such as 7-oxabicyclo[2,2,1]heptanyl.

The term **"aryl"** corresponds to a mono- or bi-cyclic aromatic hydrocarbons having from 6 to 12 carbon atoms. For instance, the term "aryl" includes phenyl, biphenyl, or naphthyl. In a preferred embodiment, the aryl is a phenyl.

The term **"aryloxy"** corresponds to the aryl group as above defined bonded to the molecule by an -O- (ether) bond. For instance, the term "aryloxy" includes phenoxy.

The term **"heteroaryl"** as used herein corresponds to an aromatic, mono- or poly-cyclic group comprising between 5 and 14 atoms and comprising at least one heteroatom such as nitrogen, oxygen or sulphur atom. Examples of such mono- and poly-cyclic heteroaryl group may be: pyridinyl, thiazolyl, thiophenyl, furanyl, pyrrolyl, pyrazolyl, imidazolyl, triazolyl, tetrazolyl, benzofuranyl, thianaphthalenyl, indolyl, indolinyl, quinolinyl, isoquinolinyl, benzimidazolyl, tetrahydroquinolinyl, tetrahydroisoquinolinyl, triazinyl, thianthrenyl, isobenzofuranyl, chromenyl, xanthenyl, phenoxanthinyl, isothiazolyl, isoxazolyl, pyrazinyl, pyridazinyl, indolizinyl, isoindolyl, indazolyl, purinyl, quinolizinyl, phtalazinyl, naphthyridinyl, quinoxalinyl, quinazolinyl, cinnolinyl, pteridinyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, indolinyl, isoindolinyl, oxazolidinyl, benzotriazolyl, benzisoxazolyl, oxindolyl, benzoxazolinyl, benzothienyl, benzothiazolyl, isatinyl, dihydropyridyl, pyrimidinyl, s-triazinyl, oxazolyl, or thiofuranyl. In a preferred embodiment, the heteroaryl group is a thiophenyl, a pyridinyl, a pyrazinyl, or a thiazolyl.

The term **"halogen"** corresponds to a fluorine, chlorine, bromine, or iodine atom, preferably a fluorine or a chlorine, and more preferably a chlorine.

The expression **"substituted or unsubstituted"** or **"optionally substituted"** means that the radical may be substituted by one or several groups of the list, or may not be substituted. Examples of the substituents which may be substituted on the aryl group, the heteroaryl group and the arylene group include a C₁₋₄ alkyl group (for example, a methyl, an ethyl, a propyl, an n-butyl, an s-butyl , a t-butyl, an isobutyl group, and the like), a halo C₁₋₄ alkyl group (for example, a chloromethyl group, a dichloromethyl group, a fluoromethyl group, a difluoromethyl group, a trifluoromethyl group, a pentachloroethyl group, and the like), a halogen atom (for example, a fluorine atom, a chlorine atom, a bromine atom, an iodine atom, and the like), C₁₋₄ alkoxy (for example, a methoxy group, an ethoxy group, a propoxy group, an isopropoxy group, an n-butoxy group, an s-butoxy group, a t-butoxy group, an isobutoxy group, and the like), or a hydroxyl group.

The expression **"radical"** or **"substituent"** refers to an alkyl, a heteroalkyl, an alkenyl, an alkynyl, an alkoxy or alkyloxy, a cycloalkyl, a heterocycloalkyl, an aryl, an aryloxy, an heteroaryl, an halogen atom, or any other group as defined above.

As used herein, the term **"treatment"** or **"therapy"** refers to any of the alleviation, amelioration and/or elimination, reduction and/or stabilization (e.g., failure to progress to more advanced stages) of a disease or a symptom thereof, as well as delay in progression of the disease, or of a symptom thereof.

The **"patient"** or **"subject"** is typically a mammal subject, preferably a human subject, or pet animal such as a dog or a cat, regardless of the age, sex, or severity of the condition.

The terms **"quantity," "amount,"** and **"dose"** are used interchangeably herein and may refer to an absolute quantification of a molecule.

### The adiponectin receptor agonists

The present invention makes use of agonists of an adiponectin receptor. The adiponectin receptor (also referred to as "AdipoR") has two subtypes, named AdipoRl and AdipoR2. AdipoRl activates the AMPK pathways and AdipoR2 activates the PPARα pathways.

The agonists useful in the present invention bind to the adiponectin receptor and activate an AMPK pathway and/or a PPARα pathway. Preferably the agonist shows a binding affinity (Kd) from 1.8x10⁻⁶ M to 3x10⁻⁶ M.

More particularly, many of the agonists useful in the present invention were described in European patent application EP3053911 for treating conditions such as type II diabetes, hypertension, lipid metabolism disorder, or obesity.

The agonists useful in the present invention are compounds of formula (1): 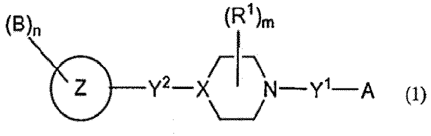 wherein
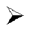 A represents a phenyl group optionally substituted with an halogen atom or a methyl group,
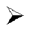 Y¹ represents - (CHR²)ₐ -, wherein R² represents H or a methyl group and a represents 1;
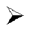 X is CH;
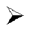 R¹ represents a C₁₋₇ alkyl group with m representing 0;
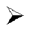 Y² represents *-O-CH₂-CONH;;
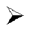 Z represents a phenyl group
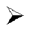 (B)ₙ represents -CO-R⁴ or -O-R⁴, wherein R⁴ is chosen from:
   - a phenyl group optionally substituted with a halogen atom, or
   - a pyridyl group optionally substituted with a halogen atom, and ;
   n represents 1
or a pharmaceutically acceptable salt thereof.

Particular embodiments of the invention are defined as in the present claims.

The following embodiments refer to the present disclosure:
In a particular embodiment, A represents a substituted or unsubstituted aryl group, or a substituted or unsubstituted heteroaryl group.

In a preferred embodiment, A represents a aryl group, preferably a phenyl group, optionally substituted with a C₁₋₆ alkyl group such as a methyl group, and/or optionally substituted with a halogen atom, preferably a chlorine or a fluorine, more preferably a chlorine.

In a particular embodiment, Y¹ represents -(CHR²)ₐ-, wherein R² represents H or a C₁₋₇ alkyl group, and a represents 1.

In a preferred embodiment, Y¹ represents -(CHR²)ₐ-, wherein R² represents a methyl group and a represents 1. According to this embodiment, Y¹ corresponds to an ethylene group (-CH-CH₃).

In a further preferred embodiment, Y¹ represents -(CHR²)ₐ-, wherein R² represents H and a represents 1. According to this embodiment, Y¹ corresponds to a methylene group (-CH₂-).

In a particular embodiment, R¹ represents a C₁₋₇ alkyl group, and m represents 0. According to this particular embodiment (R¹)ₘ does not exist since m is equal to 0.

In a preferred embodiment, X is CH.

According to this preferred embodiment, Y² represents *-O-CH₂-CONH-.

In a particular embodiment, Z represents a cyclic group selected from the group consisting of an aryl group and a heteroaryl group, preferably an aryl group. More preferably, Z represents a phenyl group.

In a particular embodiment, B represents -CO-R⁴ or -O-R⁴, wherein R⁴ is a substituted or unsubstituted phenyl group, or a substituted or unsubstituted pyridyl group.

In a preferred embodiment, B represents -O-R⁴, wherein R⁴ is an unsubstituted phenyl group or an unsubstituted pyridyl group.

In a further preferred embodiment, B represents -CO-R⁴, wherein R⁴ is a phenyl group, optionally substituted with a halogen atom, preferably a chlorine.

Preferably,n represents 1. According to this preferred embodiment, the cycle Z is monosubstituted.

In a more preferred embodiment,
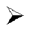 A represents a phenyl group optionally substituted with a C₁₋₆ alkyl group or a halogen atom, preferably a chlorine or a fluorine,
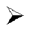 Y¹ represents - (CHR²)ₐ -, wherein R² represents H or a C₁₋₄ alkyl group, preferably a methyl group, and "a" represents 1;
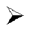 X is CH;
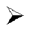 R¹ represents a C₁₋₇ alkyl group with m = 0;
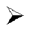 Y² represents *-O-CH₂-CONH;
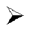 Z represents an aryl group, preferably a phenyl group
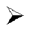 (B)ₙ represents -CO-R⁴ or -O-R⁴, wherein R⁴ is chosen from:
   - a phenyl group optionally substituted with a halogen atom, preferably a chlorine or,
   - a pyridyl group optionally substituted with a halogen atom, and
   - n represents 1.

In a preferred embodiment, the adiponectin receptor agonist useful in the present invention is selected from the following compounds:

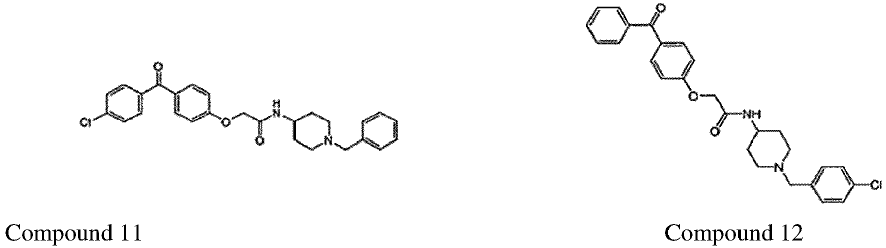

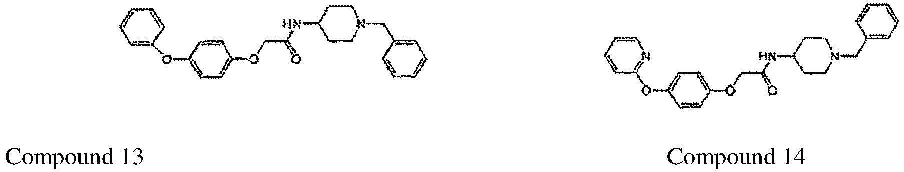

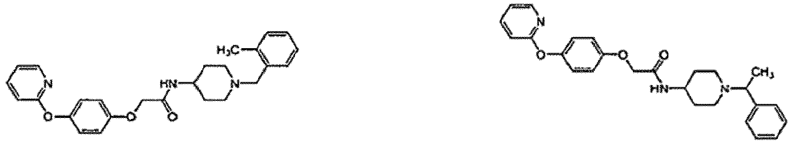

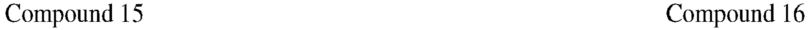

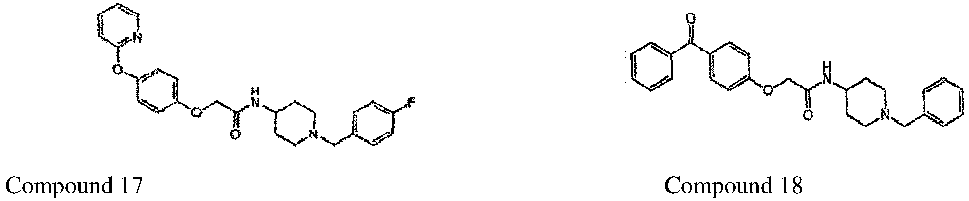

In a more preferred embodiment,
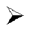 A represents a phenyl group,
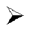 Y¹ represents - (CHR²)ₐ -, wherein R² represents H and "a" represents 1;
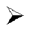 X is CH;
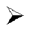 R¹ represents a C₁₋₇ alkyl group with m = 0;
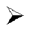 Y² represents *-O-CH₂-CONH;
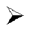 Z represents an phenyl group
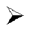 B represents -CO-R⁴ wherein R⁴ is a phenyl group, and n represents 1.

According to this preferred embodiment, the adiponectin receptor agonist for use according to the invention is 2-(4-Benzoylphenoxy)-N-[1-(phenylmethyl)-4-piperidinyl]-acetamide. This compound is also called "AdipoRon" and has the following formula:

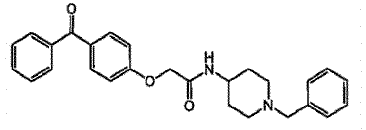

All the compounds described above can be synthesized according to the methods described in EP3053911.

### Therapeutic uses

The present disclosure provides a method for treating depression, anxiety, neuroinflammation or any neuroinflammatory disorders in a subject, comprising administering a therapeutically effective amount of an adiponectin receptor agonist, more particularly an adiponectin receptor agonist of formula (1), in a subject in need thereof.

The present disclosure further provides a use of an adiponectin receptor agonist of formula (1) for manufacturing a drug for treating depression, anxiety, neuroinflammation or any neuroinflammatory disorder.

In a first embodiment, the adiponectin receptor agonist is useful in treating depression. Depression may be a depressive episode or a recurrent or chronic depressive disorder.

A depressive episode may be a mild or a moderate depressive episode. It may also be a severe depressive episode with or without psychotic symptoms. The Diagnostic and Statistical Manual of Mental Disorders (DSM-5), American Psychiatric Association. Fifth edition. 2013, describes depression or depressive disorders in details. In typical mild, moderate, or severe depressive episodes, the patient suffers from lowering of mood, reduction of energy, and decrease in activity. Capacity for enjoyment, interest, and concentration is reduced, and marked tiredness after even minimum effort is common. Sleep is usually disturbed and appetite diminished. Self-esteem and self-confidence are almost always reduced and, even in the mild form, some ideas of guilt or worthlessness are often present. The lowered mood varies little from day to day, is unresponsive to circumstances and may be accompanied by so-called "somatic" symptoms, such as loss of interest and pleasurable feelings, waking in the morning several hours before the usual time, depression worst in the morning, marked psychomotor retardation, agitation, loss of appetite, weight loss, and loss of libido.

In another embodiment, the adiponectin receptor agonist is for use in treating anxiety, whether it is associated to a depressive disorder or not. When anxiety is linked to a depressive disorder, symptoms of anxiety and depression are both present, but neither is clearly predominant, and neither type of symptom is present to the extent that justifies a diagnosis if considered separately. Anxiety can also be linked to a phobic disorder i.e. in certain well-defined situations that are not currently dangerous. As a result these situations are characteristically avoided or endured with dread. The patient's concern may be focused on individual symptoms like palpitations or feeling faint and is often associated with secondary fears of dying, losing control, or going mad.

Contemplating entry to the phobic situation usually generates anticipatory anxiety. Phobic anxiety and depression often coexist. Examples of phobic anxiety disorders are agoraphobia, social phobias as well as specific phobias such as fear of darkness, closed spaces or certain types of animals.

In a further embodiment the adiponectin receptor agonist is for use in treating neuroinflammation, or any neuroinflammatory disorder. A neuroinflammatory disorder refers to a disease caused or associated with an inflammation of the nervous tissue of the central nervous system (CNS), including the brain, retina and spinal cord, or of the peripheral nervous system. Neuroinflammation may be chronic, or acute, it may be mild or severe and may be initiated in response to a variety of factors, including infection, traumatic brain injury, autoimmunity, depression, or a neurodegenerative disorders such as Alzheimer's disease, Parkinson's disease, multiple sclerosis, amyotrophic lateral sclerosis, frontotemporal lobar dementia or Nasu-Hakola disease (see Ransohoff 2016) as well as retinal neurodegeneration diseases.

### Administration and dosage

It is herein described a pharmaceutical composition comprising the adiponectin receptor agonist as described above. Typically, the pharmaceutical composition further comprises a pharmaceutically acceptable support.

In the context of the invention, the term "pharmaceutically acceptable support" denotes substances such as excipients, carriers, adjuvants, buffers which are conventionally used, in combination with the active ingredient(s), for the preparation of a medicament. The choice of such supports depends essentially on the route of administration envisaged.

The pharmaceutical composition preferably comprises an amount of compound according to the invention of between 5 µg and 1000 mg, preferably between 1 and 500 mg, preferably between 5 and 100 mg.

The ratio between the amounts by weight of compound according to the invention and of pharmaceutically acceptable support is between 5/95 and 95/5, preferably between 20/80 and 80/20.

The adiponectin receptor agonist may be used alone or in combination. It may be the only active ingredients, or they may be combined with other active ingredients. The pharmaceutical composition may thus also comprise at least one other pharmaceutical active agent, in particular at least one other medicament used for the treatment of depression, anxiety, or neuroinflammation.

The compounds or compositions may be administered in various ways and in various forms. Thus, they may be administered systemically, orally, by inhalation or by injection, for instance intravenously, intramuscularly, subcutaneously, transdermally, etc., intravenous, intramuscular, subcutaneous and oral administration and administration by inhalation being preferred. For the injections, the compounds are generally conditioned in the form of liquid suspensions, which can be injected by means of syringes or infusions, for example. In this regard, the compounds are generally dissolved in buffered, isotonic, physiological, saline, etc., solutions which are compatible with pharmaceutical use and known to those skilled in the art. Thus, the compositions may contain one or more agents or carriers chosen from dispersants, solubilizing agents, stabilizers, preservatives, etc. Agents or carriers which can be used in liquid and/or injectable formulations are, in particular, methylcellulose, hydroxymethylcellulose, carboxymethylcellulose, polysorbate 80, mannitol, gelatin, lactose, vegetable oils, acacia, etc.

The compounds can also be administered in the form of gels, oils, tablets, suppositories, powders, gel capsules, capsules, aerosols, etc., optionally by means of galenical forms or devices which provide prolonged and/or delayed release. For this type of formulation, an agent such as cellulose, carbonates or starches is advantageously used.

It is understood that the flow rate and/or the dose injected can be adjusted by those skilled in the art according to the patient, to the pathological condition concerned, to the mode of administration, etc. Typically, the compounds are administered at doses which can range between 0.1 µg and 100 mg/kg of body weight, more generally from 0.01 to 10 mg/kg, typically between 0.1 and 10 mg/kg. Generally, between 5 µg and 1000 mg, preferably between 1 and 500 mg, preferably between 5 and 100 mg per day are administered. In addition, repeated injections can be carried out, as appropriate. Furthermore, for chronic treatments, delayed or prolonged systems can be used.

It is understood that the dose administered can be adjusted by those skilled in the art according to the patient, to the pathological condition concerned, to the mode of administration, etc. Typically, the compounds for oral administration are at doses which can range between 0.1 and 10 mg/kg of body weight, more generally from 0.5 to 5 mg/kg, typically between 1 and 3 mg/kg, such as 1 mg, 1,5 mg, 2 mg, 2,5 mg, or 3 mg. Generally, between 0.05 and 50 mg, preferably between 0.25 and 30 mg, preferably between 1 and 10 mg per day are administered. In addition, repeated administration can be carried out, as appropriate.

A patient may be administered with the agonist of adiponectin receptor chronically, e.g. during a period of between 15 days to one year, preferably 1 month to 6 months, preferably daily, or at least once a week.

The Example and Figures illustrate the invention without limiting its scope.

### Example 1: Effect of AdipoRon on depression-like mouse models

AdipoRon, dimethylsulfoxyde (DMSO), lipopolysaccharide (LPS) from Escherichia Coli 0111:B4, bromodesoxyuridine (BrdU), paraformaldehyde (PFA), corticosterone (cortico), - cyclodextrine (-CD), bovine serum albumin (BSA) were purchased from Sigma-Aldrich.

### Mice

Male C57BL/6J mice wild type (wt) or transgenic adiponectin knock-out mice (ApN^{-/-}) were obtained from Janvier (France) and the Jackson Laboratory respectively. Mice were housed at 22°C on a 12 hour light/dark cycle (lights on/off at 7am/7pm) and allowed free access to drink and chow (A04 Safe, 2900 kcal/kg).

### Chronic and acute treatments of AdipoRon in mouse models of acute sickness and depression-like behaviors

Three well-established mouse models of depression-like behavior have been used in the present study namely i- the long-term corticosterone treatment in the drinking water (David, Samuels et al. 2009), ii- the peripheral administration of LPS (O'Connor, Lawson et al. 2009) iii- the ApN^{-/-} mice (Liu, Guo et al. 2012). For chronic AdipoRon treatment, the experimental protocol was as followed:
i- Five weeks old male mice (five/cage) received corticosterone (35mg/L dissolved in tap water containing 4 g/L CD) or vehicle alone *ad libitum* for during 7 consecutive weeks (David, Samuels et al. 2009). Corticosterone and vehicle solutions were changed every 3 days to prevent possible degradation. Additionally, during the last three weeks of corticosterone treatment, mice were daily intraperitoneally (*ip*) injected with AdipoRon (1mg/kg in 2.5% DMSO) or vehicle alone.
ii- Eight weeks old male mice daily *ip* injected with AdipoRon or vehicle as described above for twenty consecutive days. One day prior behavioral testing, mice were injected *ip* with LPS (0.83 mg/kg) or saline solution; the LPS dose was selected on the basis of its ability to induce the full spectrum of the acute sickness and depressive-like behaviors (Lestage, Verrier et al. 2002).

### Behavioral testing

All behavioral testing were conducted during the light phase (between 09:00 and 14:00 am) in compliance with the Institutional Animal Care and Use Committee of the University of Nice-Sophia Antipolis (permission number 010344.01 from the French "Ministère de l'Enseignement Supérieur et de la Recherche" granted to Dr. Joëlle Chabry).
***Forced Swimming test (FST)*** - Mice were placed an inescapable transparent tank (20cm diameter, 30cm deep) filled with water (23°C ± 0.5) for six minutes as previously described by Porsolt et al. (1977). The immobility duration, as an index of the resignation, was timed during the last four minutes of the test. A mouse was considered immobile when it remained floating with only slight movements to keep its head out of the water.
***Open-field test (OF)*** - Locomotor activity was determined using open-field (OF) activity. Mice were placed in the corner of the test apparatus (45Lx45Wx25H cm Plexiglas box) with free moving for 10 minutes; the entire sessions were videotaped. The automated analysis done using the ANY-maze software (ANY-maze© version 4.6, Stoelting Co., USA) resulted in a track record of the mouse movements in the open field arena allowing evaluation of locomotor activity i.e. total distance traveled and mean speed.
***Rotarod*** - Mice were placed on a rotating for two 5 min-habituation phases on fixed rod (10 rpm/min) four hours apart. Twenty-four hours later, the latency to fall, an index of the motor coordination, was recorded on accelerating rod (from 10 to 40 rpm/min).
***Light & Dark test (L&D)* -** The L&D place preference test was used to assess the anxiety-like behavior. The test apparatus (40Lx30Wx25H cm) was divided into equal zones (i.e., light or dark zones) with a doorway connecting the two sides. The light zone was very bright (600 lux) while the dark zone was protected from light by an opaque lid. To initiate testing, mice were placed into the light side and activity was recorded for 5 minutes. Anxious mice entered the light zone seldom and spent less time in it.
***Sucrose preference*** - Mice were habituated to two water bottles twenty-four hours before test. For the test a bottle of water and a bottle of 1% sucrose solution were given to the mice. The amount of water and 1% sucrose solution was measured twenty-four hours later. The sucrose preference rate = (Sucrose consumption/(water consumption+ sucrose consumption))* 100 ***Novelty Suppressed Feeding (NSF)* -** NSF assesses stress-induced anxiety by measuring the latency of an animal to approach and eat a familiar food in an aversive environment. This test is a conflict test that elicits competing motivations: the drive to eat and the fear of venturing into the center of the brightly lit arena (500-600 lux). The testing apparatus consisted of a plastic box, the floor of which was covered with approximately 2 cm of wooden bedding. Twenty hours prior to behavioral testing, all food was removed from the home cage. At the time of testing, a single pellet of food was placed on a Plexiglass platform in the center of the box. An animal was placed in a corner of the box, and the entire session was videotaped (i.e. 10 minute period). The latency to eat (defined as the mouse sitting on its haunches and biting the pellet) was timed. Immediately after the test, food consumption was measured for 30 min as control for potential feeding differences.
***Social interaction test*** - The social testing apparatus was a rectangular, three-chambered box with clear Plexiglas walls, having small circular openings (3.5 cm in diameter) allowing access into each chamber (25Lx15Wx20H cm). Three interconnected chambers are separated by manually operated sliding doors. The test mouse was first placed in the middle chamber and allowed to explore the entire apparatus for a five-min habituation period. At the end of the session, the test mouse was confined into the centre chamber for two min by obstruction of the doorways between the two side chambers. A wire cup-like container was placed in the corner of each chamber; one is empty (*right chamber*), in the second (*left chamber*) was placed an unfamiliar mouse with same genotype, sex and age range. Both doors to the side chambers were then unblocked and the test mouse was allowed to freely explore the entire test box for a 5-min session. The time spent in the right (*empty*) and left (*stranger mouse*) chamber was timed allowing the assessment of the interaction with an animate object *versus* sociability (Moy, Nadler et al. 2004).
***Learned Helplessness (LH)*** - Mice were then placed into the shock chamber and exposed to inescapable shocks comprised 360 scrambled footshocks (intensity: 0.3 mA) with 2-sec duration and an interval-episode of 10-sec, amounting to total session duration of approximately 60-min. The shock procedure was repeated four consecutive days. One day after the last shock procedure, learned helplessness was assessed by testing shuttle box performance. Each trial started with a 0.3 mA footshock of maximum 24-sec duration. The inter-trial interval was 60-sec. The following behavioral reactions were defined: escape as shuttling to the other compartment as reaction to the electric shock, and failure when no attempt to escape was made. Furthermore, the parameter escape latency was recorded as the time needed to shuttle into the other compartment after onset of footshocks. The mice that failed to escape were excluded from the escape latency measurement. Total time of testing for helplessness lasted about 20-24 minutes, the exact time period depending on the animal's ability to learn the paradigm and to respond properly. Control animals underwent the same handling and contextual procedures without receiving the footshocks. Before each trial, the apparatus was thoroughly cleaned with 70% ethanol and dried.
***Hippocampal neurogenesis*** - Mice were *ip* injected with BrdU (50 mg/kg of body weight) once a day for five consecutive days, a week prior the onset of the chronic treatment with AdipoRon. Twenty-one days after the last BrdU injection, mice were euthanized and transcardially perfused with cold PBS then fixed with 3.2% PFA. Serial coronal sections of the brains were cut (40 µm) throughout the hippocampus (from bregma 3.3 to 5.3) on a vibratome (Leica). Eight sections (one every fifth section) throughout the hippocampus were processed for BrdU immunohistochemistry as follows. Slides were first incubated for one hour at room temperature in PBS containing 2.5% horse serum then overnight at 4°C with a mouse monoclonal anti-BrdU antibody (1:7 500; Becton-Dickinson) and finally for 2 hours in biotin-conjugated species-specific secondary antibodies (1:400; Vector Laboratories) followed by a peroxidase-avidin complex solution according to the manufacturer's protocol. The peroxidase activity of immune complexes was visualized with DAB staining using the VectaStain ABC kit (Vector Laboratories). BrdU-labeled cells in the subgranular layer (SGL) and granule cell layer (GCL) were counted in each section at 40x magnification under a light microscope (Olympus). The total number of BrdU-positive cells per eight slices was multiplied by five to obtain the total number of cells per dentate gyrus.
***Kinetic studies of Adiporon effects*** - Ten to fourteen week-old male C57BL/6J mice were *ip* injected with AdipoRon (1mg/kg) or vehicle alone (2.5% DMSO). At different time point posttreatment (0, 30 min, 1h, 3h, 6h), mice were anesthetized by lethal *ip* injection of sodium pentobarbital; blood samples were collected then perfusions were performed by intracardiac puncture of 30ml of cold saline solution following by dissection of various brain areas. Samples were stored at -80°C until use. Alternatively, saline solution perfusions were followed by fixation with 3.2% PFA; then brains were collected and preceded for immunochemistry studies.
***Acute brain slices* -** Mice were deeply anesthetized with halothane, decapitated and brains were immediately placed into ice-cold gassed medium (95% O₂/5% CO₂) containing (in mM): 125 NaCl, 2.5 KCl, 1 MgCl₂, 0.4 CaCl₂, 1.25 NaH₂PO₄, 26 NaHCO₃ and 25 glucose. Coronal slices at the level of the fourth ventricle (350µM thick) were cut with an HM650V vibratome (Microm, Walldorf, Germany) and placed in a holding chamber at 34 °C for 1 h. Slices were then transferred ar room temperature in Phosphate Bicarbonate Buffer Saline (PBBS) composed of (in mM): 125 NaCl, 2.5 KCl, 1 MgCl₂, 2 CaCl₂, 1.25 NaH₂P0₄, 26 NaHC0₃ and 25 glucose, pH 7.4 when bubbled with 95% O₂/5% CO₂.
***Whole-cell patch-clamp recordings* -** Brain slices containing the raphe nucleus were placed under a Nomarski microscope (Zeiss, France) equipped with infrared video camera (Axiocam, Zeiss, France) in a recording chamber superfused at a flow rate of 1 ml.min⁻¹ with oxygenated PBBS (2mM CaCl₂). Recordings from median and dorsal raphe nucleus neurons were made at room temperature (25 ± 2°C) using a Axopatch 200B (Axon Instruments, USA). Patch clamp pipettes made from borosilicate glass capillary (Hilgenberg, Germany) had a resistance of 3-8 MΩ when filled with the internal solution containing (mM): 130 Kgluconate, 1 MgCl₂, 0.3 CaCl₂, 1 EGTA, 4 Mg2ATP, 0.4 Na3GTP, 10 HEPES (pH adjusted to 7.3 with KOH). Neurons were first patch-clamped in cell-attached mode to record spontaneous action potential firing and then in the whole-cell configuration in current clamp. Values of access resistance ranged from 12 to 20 MΩ and were not compensated. Measurements were made 2-3 min after obtaining the whole-cell to ensure dialysis. Cell capacitance and resistance were measured in voltage clamp using the pClamp Clampex software by applying 5 mV voltage steps. Solutions were applied in the bath. Current-clamp recordings were made in the IClamp mode of the Axopatch 200B. Serotoninergic neurons were recognized on the basis of their regulatory action potential discharge at a low frequency and their hyperpolarizing response to the application of 5-HT.

Voltage-clamp experiments were performed with a KCl solution of the following composition: 390 KCl, 5 MgCl₂, 1 CaCl₂, 10 Hepes, 10 EGTA, 4 Na₂ATP, 0.4 Na₃GTP, pH 7.3, 300 mOsm). Membrane conductance was evaluated by applying small pulses of -10 mV and 200 ms every 10 sec.

Data were digitized at 5-10 kHz using a Digidata interface coupled to a microcomputer running pClamp 9 (Axon Instruments, USA). Current-clamp data were digitized at 0.5 kHz using the same interface. Potentials were digitally filtered at 1-3 kHz. Average data were expressed as mean ± SEM, n = number of neurons, N = number of mice.
***Western blot analysis*** - Total proteins were extracted from mouse hypothalamus by homogenization in lysis buffer (50mM Tris-HCl, pH7.5, 150mM NaCl, 5mM EDTA containing 0.5% Triton X-100 and 0.5% sodium deoxycholate with a coktail of protease and phosphatase inhibitors). Equal amounts of total proteins (50µg) determined by the Bradford method (BioRad) were separated onto 12% SDS-PAGE, then transferred on nitrocellulose membrane (Schleicher & Schuell, Dassel, Germany). Blots were incubated in PBS 0.1% Tween, 2% BSA with antibodies directed against phospho-AMPKα (Thr172), total AMPK (Cell Signaling), and then with the appropriate HRP-secondary antibody. Blots were developed using an enhanced chemoluminescence system (Immobilon, Merck Millipore) with a Fusion detector (Vilber). To correct for any loading artifact, same blots were re-probed with anti-actin antibody (Abcam). Densitometry analyses were performed with a "National Institutes of Health" Image software™ on the immuno-positive bands.
***Cytokines measurement*** - Hypothalamus, hippocampus and prefrontal cortex were homogenized in lysis buffer (10mM Tris-HCl, pH7.5, 150mM NaCl, 5mM EDTA containing 10% Glycerol and 1% NP-40 with protease inhibitor). IFNγ, IL1β, IL6, and TNFα, levels were measured using an electro-chemo-luminescence (ECL)-based assay (U-plex, MesoScaleDiscovery) following manufacturer instructions. Results were standardized according to the protein concentration of each sample determined by the Bradford method.
***Kynurenine pathway metabolites measurement*** - Plasma levels of L-kynurenin (L-Kyn), tryptophan (Trp), kynurenin acid (Kyn ac), 5-HT and 5-HIAA were determined as follows: 25µl of plasma was deproteinated with 150µl of cold MeOH. Weighted brain tissues were mechanically homogenized in 100µl of 0.2% formic acid, incubate in ice for 15min then centrifuged at 4000 rpm for 5min at 4°C. 300µl of cold MeOH were added to supernatants and methanol precipitation was pursed for 30min at -20°C, then samples were centrifuged at 10,000 x g for 10 min at 4°C; supernatants were dried under speed vacuum centrifuge then resuspended in 50 µl of the mix 90% water, 10% acetonitrile, 0.1% formic acid. Determination of L-Kyn, Trp, Kyn ac, 5-HT and 5-HIAA levels, done in triplicate, were determined by liquid chromatography coupled to MS-MS analysis as previously described with minor modifications (Fuertig, Ceci et al. 2016).
***Statistical Analysis*** - Statistical analyses were performed using GraphPad Prism 4.0 software. Biological parameters and q-PCR data were analyzed using a one-way (treatment) or two-way (pretreatment X treatment) analysis of variance, followed by a post-hoc multiple comparison procedure using the Dunnett's method. Behavioral data were analyzed using the nonparametric Kruskal-Wallis test and when warranted, *post hoc* Dunn's comparisons were carried out. Comparisons between two conditions were made using the nonparametric Mann & Whitney test. Data are presented as means ± standard error of the mean (SEM); Statistical significance was set at **P*<0.05 and ***P*<0.01, ****P*<0.001.

### RESULTS

### Effects of acute administration of AdipoRon on various depression-like mouse models

The FST is the gold standard rodent behavioral test for evaluation of antidepressant efficacy of drugs and high throughput screening of new compounds. To evaluate the antidepressant potential of AdipoRon, adult C57BL/6J mice were *ip* injected with AdipoRon (1mg/kg) or vehicle alone then submitted to the FST 1h or 3h later on. AdipoRon decreased the immobility time of mice when measured 1h post-injection compared to vehicle while no significant effect was observed 3h post-injection (fig 1A). Taking account of the observed transient effect of acute administration of AdipoRon, the following experiments were performed 1h post-ip injection of AdipoRon. To define the optimal dose of AdipoRon able to decrease the immobility duration in the FST, dose-response experiments were performed on wt (fig 1B) and depression-like mouse models namely corticosterone-treated (fig 1C), ApN^{-/-} (fig ID) and LPS-treated mice (fig IE). One-hour post injection, 0.5mg/kg of AdipoRon significantly reduced the immobility time of wt and corticosterone-treated mice (fig 1B, C) while this dose remained inefficient to produce an effect on ApN^{-/-} mice (fig ID). 5mg/kg of AdipoRon decreased the immobility time of wt, corticosterone-treated and ApN^{-/-} and 1mg/kg of AdipoRon was enough to significantly to reducing this parameter in LPS-treated mice (fig IE). Based on these results, the dose of AdipoRon of 1 mg/kg was chosen to perform all the subsequent experiments. Together, these results allowed us to determine a dose of AdipoRon of 1 mg/kg. Then we investigated whether an alternative administration of AdipoRon i.e. *per os* led to a reduction of the immobility time in the FST. To do so, wt or ApN^{-/-} mice were gavaged with AdipoRon (1mg/kg) or vehicle alone, then submitted to the FST one-hour later one (fig IF, G). AdiopoRon administrated *per os* reduced the immobility time in both wt and ApN^{-/-} mice.

In summary, a single *ip* or *per os* administration of AdipoRon produced a transient reduction of the « despair-like » behavior in depression-like mice.

### Effects of chronic administration of AdipoRon on a depression-like mouse model based on long-term corticosterone exposure

We sought to investigate the potential beneficial effects of chronic administration of Adiporon on anxiety and depression-like behaviors (fig 2A). First, the anxiety behavior of mice was assessed using the light-dark paradigm (L&D). As expected, corticosterone-treated mice exhibited reduced time spent in the aversive light chamber of the LD compared to the vehicle-treated mice (fig. B). Interestingly, chronic administration of AdipoRon reversed the anxiety phenotype in corticosterone-treated group while it remained without effect on vehicle-treated mice. As previously described (Nicolas et al. 2015; David et al. 2009), long-term corticosterone treatment promoted a depression-like behavior in mice as assessed using behavioral tests aiming at measuring anhedonia (sucrose reference test, fig 2C), resignation (FST, fig 2D), anxio-depressive-like behavior (NSF, fig 2E) social interactions and learned helplessness (fig 2G, H, I). Briefly, long-term exposure to corticosterone reduced the sucrose preference, increased the immobility duration and the latency to eat in the FST and in the NSF respectively, lowered social interactions and favored the resignation-like behavior in the LH test. Interestingly, chronic administration of AdipoRon reversed the effect of corticosterone in all the cited paradigms whereas no significant effect was observed in vehicle-treated mice (fig 2C, D, E, F, G, H, I). In adipo^{-/-} mice, EE housing did not significantly modify the OF parameters namely the time spent, the number of entries and the latency to first entry in the aversive center of the arena (Fig.3A). Altogether, our data suggest that chronic treatment of AdipoRon prevented anxiety and depression-like behavior in a mouse model of depression.

Note that chronic AdipoRon treatment did not significantly modify the weight of mice (fig 3D), food and water intake, locomotion parameters assessed in the OF test (i.e total distance traveled and mean speed, (fig 3A, B) nor the motor coordination assessed using the rotarod apparatus (fig 3C).

### AdipoRon reverses the deleterious effect of long-term corticosterone treatment on hippocampal neurogenesis

Because antidepressant drugs like fluoxetine or imipramine promote hippocampal neurogenesis on adult brain, "the neurogenic hypothesis" currently prevails in the depression research field. To further inside the potential cellular mechanisms underlying the benefits of AdipoRon on depression, we evaluated changes in adult hippocampal neurogenesis and neuronal survival (assessed 21-days post-BrdU injections, fig 4). Long-term corticosterone exposure decreased the hippocampal neurogenesis rate while AdipoRon reversed the deleterious effect of corticosterone (fig 4B). AdipoRon treatment counteracted adverse effects of corticosterone on hippocampal neurogenesis without modifying neurogenesis in control conditions.

### AdipoRon crosses the blood-brain barrier (BBB) and targets the brain

Next, we investigated whether AdipoRon crosses the blood-brain barrier and triggers activation of intracellular signaling pathways. Plasma samples and brain areas of interest (hypothalamus, hippocampus) were collected from mice, at different time point after who *ip* injection of AdipoRon. The kinetic of BBB crossing and the recovery rate of AdipoRon in the plasma (fig 5A) and central nervous system (fig 5B,C) were determined by HPLC coupled to MS/MS analysis. The highest level of AdipoRon was measured in plasma, the hypothalamus and the hippocampus thirty min post *ip* injection, then it decreased until being barely detectable 3-hours post injection.

AdipoRon has been shown to bind both adiponectin receptors, AdipoRl and AdipoR2, both expressed in the central nervous system (Okada-Iwabu, Yamauchi et al. 2013). AdipoRl triggers AMPK pathway activation while AdipoR2 activates the PPARγ pathways. To investigate if AdipoRon targets hypothalamus, the rate of AMPK phosphorylation was assessed by western blotting at different time point post *ip* injection of AdipoRon. Interestingly, AdipoRon increased the level of phospho-AMPK 30 min and 1-hour post *ip.* Injection; these effects are no longer observable 3 hours post *ip* injection (fig 5D, E). These results provide strong evidence that AdipoRon crosses the BBB and targets the brain by binding to AdipoRl and activating related intracellular signaling pathway.

### Chronic AdipoRon treatment prevents increase of plasma kynurenine induced by long-term corticosterone exposure

A relationship between depressive symptoms and plasma level of Trp, L-KYN and Kyn ac has been established. L-Kyn:Trp and Kyn ac:L-Kyn ratio (fig 6), index of the indoleamine 2,3-dioxygenase (IDO) and kynurenine aminotransferase (KAT) enzyme activities, are therefore considered as reliable biomarkers of depression related to chronic low grade inflammation (Dantzer, O'Connor et al. 2011). As expected, long-term corticosterone triggered an increase concentration in plasma concentration of L-Kyn resulting in a Kyn ac:L-Kyn decrease (fig 6A) concomitantly to a L-Kyn:Trp increase (fig 6B). Chronic AdipoRon treatment significantly counteracted this effect, restoring the physiological L-Kyn:Trp and Kyn ac:L-Kyn ratio.

### AdipoRon increases serotonin turnover in raphe nuclei

The "serotonin hypothesis" postulates that diminished activity of serotonin pathway plays a causal role in pathophysiology of depression. Here, to investigate if AdipoRon influenced serotonin pathway, we examined the level of serotonin (5-HT) and its metabolite 5-hydroxyindoleacetic acid (5-HIAA) in the dorsal raphe nucleus from corticosterone-exposed mice chronically treated with or not AdipoRon. Although long-term corticosterone administration *per se* had no effect on 5-HT and 5-HIAA concentrations in the dorsal raphe (fig 6C, D); AdipoRon increased the level of 5-HIAA without modifying the 5-HT level resulting in a threefold increase of the ratio 5-HIAA/5-HT (fig 6E). These results suggest that AdipoRon increased the serotonin release and turn over in raphe nucleus from depressive-like mice.

### AdipoRon prevents corticosterone-induced neuroinflammation

We investigated the levels of pro-inflammatory cytokines (IL1β, IL6, TNFα, IFNγ) in various brain areas involved in depression physiopathology i.e. hypothalamus, hippocampus and prefrontal cortex) of chronically treated mice by corticosterone and adipoRon (fig 7). Hypothalamic levels of IL1β, TNFα, and IFNγ, but not IL6, were found in corticosterone-treated mice (fig 7A). Only IFNγ was increased by corticosterone-treatment in mice hippocampus (fig 7B). Prefrontal cortex of corticosterone-treated mice showed increased levels of IL1β, IL6 and TNFα, but not IFNγ. These cytokines differences were abolished by the chronic treatment with AdipoRon (fig 7C). Altogether, our results showed that AdipoRon reduced the expression of pro-inflammatory cytokines in different brain area of depressive-like mice.

### AdipoRon depolarizes presumed serotoninergic neurons of dorso-median raphe nucleus in mouse brain slices

To explore whether AdipoRon exerts of a direct action on serotoninergic neurons, we performed patch-clamp experiments on brain slices containing the dorso-median raphe, in the whole-cell mode. In 6 out of 7 presumed serotoninergic neurons recorded in current clamp in the dorso-median raphe (N= 4 mice, identified on the basis of their shape, their low frequency regular pattern of action potential discharge and the hyperpolarization of their membrane potential observed in response to the application of 5-HT at the end of the recording), AdipoRon induced an increase in action potential discharge frequency that was accompanied by a small depolarization (fig 8A). In 1/7 presumed serotoninergic neuron, AdipoRon had no effect (fig 8B). Interestingly, on neurons that were presumed non-serotoninergic neurons, AdipoRon induced a hyperpolarization (2/4, fig 8C) or had no effect (2/4).

To decipher the mechanisms of action of AdipoRon, we made voltage-clamp experiments at a holding potential of -60 mV. AdipoRon induced a small inward current (fig 8D, E) in all presumed serotoninergic neurons (i.e. raphe neurons in which 5-HT induced an outward current, n=9/9, N=5 mice), that was accompanied by a decrease in membrane conductance (fig 8F) suggesting that the outward current was due to the closing of background potassium channels opened at -60 mV in control conditions. Indeed, this effect of AdipoRon was blocked when we used a CsCl internal solution instead of a KCl solution.

### REFERENCES

Chabry, J., S. Nicolas, et al. (2015). "Enriched environment decreases microglia and brain macrophages inflammatory phenotypes through adiponectin-dependent mechanisms: Relevance to depressive-like behavior." Brain Behav Immun 50: 275-287.
Dantzer, R., J. C. O'Connor, et al. (2011). "Inflammation-associated depression: from serotonin to kynurenine." Psychoneuroendocrinology 36(3): 426-436.
David, D. J., B. A. Samuels, et al. (2009). "Neurogenesis-dependent and -independent effects of fluoxetine in an animal model of anxiety/depression." Neuron 62(4): 479-493.
Fuertig, R., A. Ceci, et al. (2016). "LC-MS/MS-based quantification of kynurenine metabolites, tryptophan, monoamines and neopterin in plasma, cerebrospinal fluid and brain." Bioanalysis 8(18): 1903-1917.
Lestage, J., D. Verrier, et al. (2002). "The enzyme indoleamine 2,3-dioxygenase is induced in the mouse brain in response to peripheral administration of lipopolysaccharide and superantigen." Brain Behav Immun 16(5): 596-601.
Liu, J., M. Guo, et al. (2012). "Adiponectin is critical in determining susceptibility to depressive behaviors and has antidepressant-like activity." Proc Natl Acad Sci U S A 109(30): 12248-12253.
Moy, S. S., J. J. Nadler, et al. (2004). "Sociability and preference for social novelty in five inbred strains: an approach to assess autistic-like behavior in mice." Genes Brain Behav 3(5): 287-302.
Nicolas, S., Veyssière, J., et al (2015). "Neurogenesis-independent antidepressant-like effects of enriched environment is dependent on adiponectin. Psychoneuroendocrinology 57 : 72-83.
O'Connor, J. C., M. A. Lawson, et al. (2009). "Lipopolysaccharide-induced depressive-like behavior is mediated by indoleamine 2,3-dioxygenase activation in mice." Mol Psychiatry 14(5): 511-522.
Okada-Iwabu, M., T. Yamauchi, et al. (2013). "A small-molecule AdipoR agonist for type 2 diabetes and short life in obesity." Nature 503(7477): 493-499
Ransohoff, R. (2016) "How neuroinflammation contributes to neurodegeneration", Science, 353 (6301):777-783

## Claims

1. An adiponectin receptor agonist for use in treating depression, anxiety or a neuroinflammatory disorder in a patient, wherein the adiponectin receptor agonist is a compound of the following formula (1): 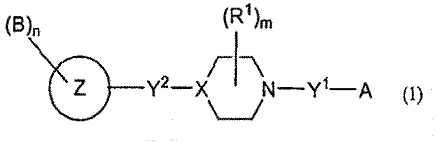 wherein:
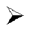 A represents a phenyl group optionally substituted with an halogen atom or a methyl group,
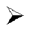 Y¹ represents - (CHR²)ₐ -, wherein R² represents H or a methyl group and a represents 1; > X is CH;
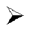 R¹ represents a C₁₋₇ alkyl group with m representing 0;
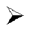 Y² represents *-O-CH₂-CONH;;
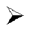 Z represents a phenyl group
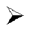 (B)ₙ represents -CO-R⁴ or -O-R⁴, wherein R⁴ is chosen from:
• a phenyl group optionally substituted with a halogen atom, or
• a pyridyl group optionally substituted with a halogen atom, and ;
n represents 1.

2. The adiponectin receptor agonist for use according to claim 1, wherein A represents a phenyl group.

3. The adiponectin receptor agonist for use according to claim 1 to 2, wherein Y¹ represents - (CHR²)ₐ -, wherein R² represents H and a represents 1.

4. The adiponectin receptor agonist for use according to claim 1 to 3, wherein (B)ₙ represents -COR⁴, wherein R⁴ is a phenyl group optionally substituted with a halogen atom, preferably a chlorine, and n represents 1.

5. The adiponectin receptor agonist for use according to claim 1 to 3, wherein (B)ₙ represents -O-R⁴, wherein R⁴ is chosen from:
• a phenyl group, or
• a pyridyl group.
and n represents 1.

6. The adiponectin receptor agonist for use according to claim 1 to 5, wherein said adiponectin receptor agonist is selected from the following compounds:

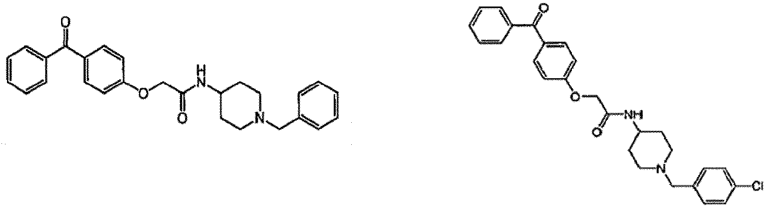

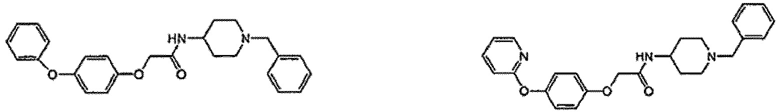

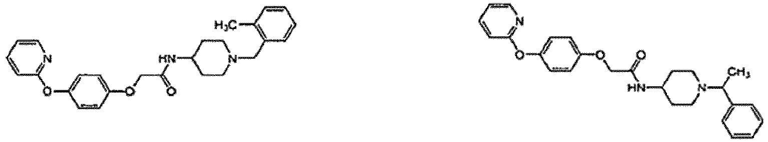

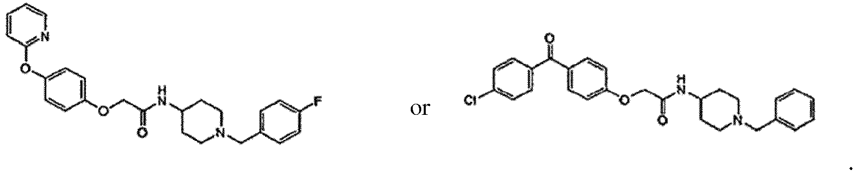

7. The adiponectin receptor agonist for use according to claim 1 to 6, wherein said adiponectin receptor agonist is 2-(4-Benzoylphenoxy)-N-[1-(phenylmethyl)-4-piperidinyl]-acetamide.

8. The adiponectin receptor agonist for use according to claim 1 to 7, for treating depression.

9. The adiponectin receptor agonist for use according to claim 1 to 7, for treating anxiety.

10. The adiponectin receptor agonist for use according to claim 1 to 7, for treating a neuroinflammatory disorder.

11. The adiponectin receptor agonist for use according to claim 10, wherein the neuroinflammatory disorder is a neurodegenerative disease with neuroinflammation.

12. The adiponectin receptor agonist for use according to claim 11, wherein the neurodegenerative disease is selected from the group consisting of Alzheimer's disease, Parkinson's disease, multiple sclerosis, amyotrophic lateral sclerosis, frontotemporal lobar dementia and Nasu-Hakola disease.

13. The adiponectin receptor agonist for use according to claim 1 to 12, wherein the compound is to be orally administered.

## Patentansprüche

1. Adiponectin-Rezeptor-Agonist zur Verwendung bei der Behandlung von Depression, Angst oder einer neuroinflammatorischen Störung bei einem Patienten, wobei der Adiponectin-Rezeptor-Agonist eine Verbindung der folgenden Formel (1) ist: 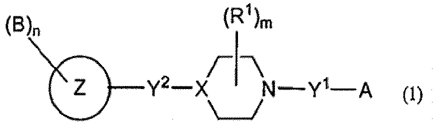 wobei:
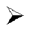 A eine Phenylgruppe darstellt, die optional mit einem Halogenatom oder einer Methylgruppe substituiert ist,
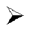 Y¹-(CHR²)ₐ- darstellt, wobei R² H oder eine Methylgruppe darstellt und a 1 darstellt;
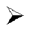 X CH ist;
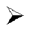 R¹ eine C₁₋₇-Alkylgruppe darstellt, wobei m 0 darstellt;
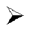 Y²*-O-CH₂-CONH darstellt;
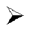 Z eine Phenylgruppe darstellt
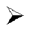 (B)ₙ -CO-R⁴ oder -O-R⁴ darstellt, wobei R⁴ ausgewählt ist aus:
• einer Phenylgruppe, die optional mit einem Halogenatom substituiert, oder
• einer Pyridylgruppe, die optional mit einem Halogenatom substituiert ist, und;
n 1 darstellt.

2. Adiponectin-Rezeptor-Agonist zur Verwendung nach Anspruch 1, wobei A eine Phenylgruppe darstellt.

3. Adiponectin-Rezeptor-Agonist zur Verwendung nach einem der Ansprüche 1 bis 2, wobei Y¹ für -(CHR²)ₐ- steht, wobei R² H darstellt und a 1 darstellt.

4. Adiponectin-Rezeptor-Agonist zur Verwendung nach einem der Ansprüche 1 bis 3, wobei (B)ₙ -CO- R⁴ darstellt, wobei R⁴ eine Phenylgruppe ist, die optional mit einem Halogenatom, vorzugsweise einem Chloratom, substituiert ist, und n 1 darstellt.

5. Adiponectin-Rezeptor-Agonist zur Verwendung nach einem der Ansprüche 1 bis 3, wobei (B)ₙ -O-R⁴ darstellt, wobei R⁴ ausgewählt ist aus:
• einer Phenylgruppe, oder
• einer Pyridylgruppe
und n 1 darstellt.

6. Adiponectin-Rezeptor-Agonist zur Verwendung nach einem der Ansprüche 1 bis 5, wobei der Adiponectin-Rezeptor-Agonist aus den folgenden Verbindungen ausgewählt ist:

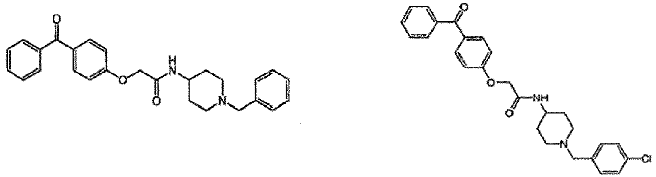

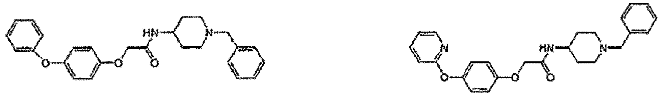

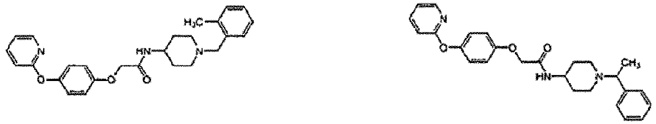

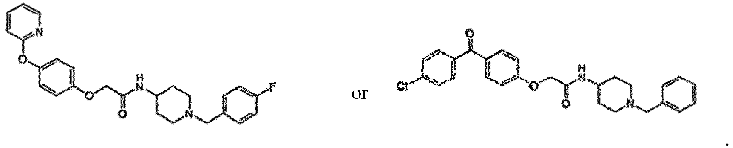

7. Adiponectin-Rezeptor-Agonist zur Verwendung nach einem der Ansprüche 1 bis 6, wobei der Adiponectin-Rezeptor-Agonist 2-(4-Benzoylphenoxy)-N-[1-(Phenylmethyl)-4-Piperidinyl]-Acetamid ist.

8. Adiponectin-Rezeptor-Agonist zur Verwendung nach einem der Ansprüche 1 bis 7 zur Behandlung von Depressionen.

9. Adiponectin-Rezeptor-Agonist zur Verwendung nach einem der Ansprüche 1 bis 7, zur Behandlung von Angststörungen.

10. Adiponectin-Rezeptor-Agonist zur Verwendung nach einem der Ansprüche 1 bis 7 zur Behandlung einer neuroinflammatorischen Störung.

11. Adiponectin-Rezeptor-Agonist zur Verwendung nach Anspruch 10, wobei die neuroinflammatorische Störung eine neurodegenerative Erkrankung mit Neuroinflammation ist.

12. Adiponectin-Rezeptor-Agonist zur Verwendung nach Anspruch 11, wobei die neurodegenerative Erkrankung ausgewählt ist aus der Gruppe bestehend aus Alzheimer-Krankheit, Parkinson-Krankheit, Multipler Sklerose, Amyotropher Lateralsklerose, Frontotemporal-Lobar-Demenz und Nasu-Hakola-Krankheit.

13. Adiponectin-Rezeptor-Agonist zur Verwendung nach einem der Ansprüche 1 bis 12, wobei die Verbindung oral zu verabreichen ist.

## Revendications

1. Agoniste du récepteur d'adiponectine pour une utilisation dans le traitement de la dépression, de l'anxiété ou d'un trouble neuroinflammatoire chez un patient, dans lequel l'agoniste du récepteur d'adiponectine est un composé de formule (I) suivante : 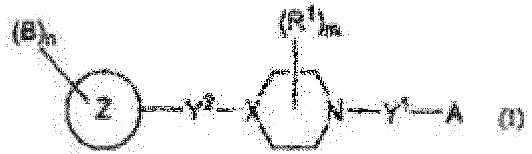 dans lequel :
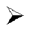 A représente un groupe phényle éventuellement substitué par un atome d'halogène ou un groupe méthyle,
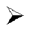 Y¹ représente -(CHR²)ₐ-, dans lequel R² représente H ou un groupe méthyle et a représente 1 ;
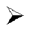 X est CH ;
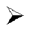 R¹ représente un groupe alkyle en C₁₋₇, m représentant 0 ;
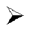 Y² représente *-O-CH₂-CONH ;
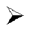 Z représente un groupe phényle
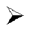 (B)ₙ représente -CO-R⁴ ou -O-R⁴, dans lequel R⁴ est choisi parmi :
• un groupe phényle éventuellement substitué par un atome d'halogène, ou
• un groupe pyridyle éventuellement substitué par un atome d'halogène, et ;
n représente 1.

2. Agoniste du récepteur d'adiponectine pour une utilisation selon la revendication 1, dans lequel A représente un groupe phényle.

3. Agoniste du récepteur d'adiponectine pour une utilisation selon la revendication 1 ou 2, dans lequel Y¹ représente -(CHR²)ₐ-, dans lequel R² représente H et a représente 1.

4. Agoniste du récepteur d'adiponectine pour une utilisation selon les revendications 1 à 3, dans lequel (B)ₙ représente -CO-R⁴, dans lequel R⁴ est un groupe phényle éventuellement substitué par un atome d'halogène, de préférence un chlore, et n représente 1.

5. Agoniste du récepteur d'adiponectine pour une utilisation selon les revendications 1 à 3, dans lequel (B)ₙ représente-O-R⁴, dans lequel R⁴ est choisi parmi :
• un groupe phényle, ou
• un groupe pyridyle,
et n représente 1.

6. Agoniste du récepteur d'adiponectine pour une utilisation selon les revendications 1 à 5, dans lequel ledit agoniste du récepteur d'adiponectine est choisi parmi les composés suivants : 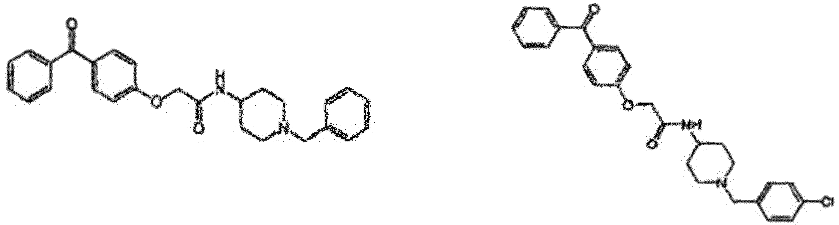 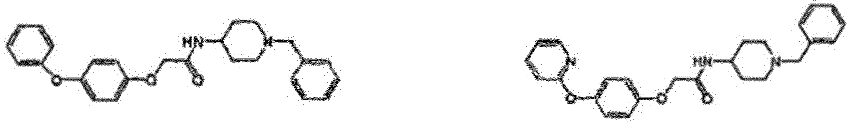 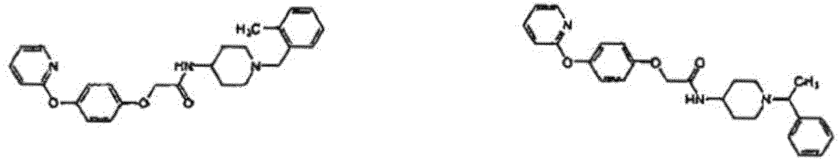 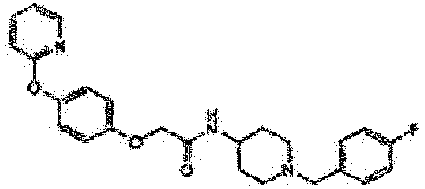 ou

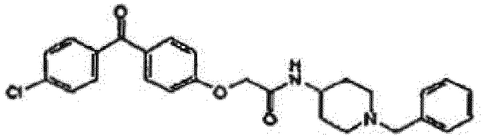

7. Agoniste du récepteur d'adiponectine pour une utilisation selon les revendications 1 à 6, dans lequel ledit agoniste du récepteur d'adiponectine est le 2-(4-benzoylphénoxy)-N-[1-(phénylméthyl)-4-pipéridinyl]-acétamide.

8. Agoniste du récepteur d'adiponectine pour une utilisation selon les revendications 1 à 7, pour traiter la dépression.

9. Agoniste du récepteur d'adiponectine pour une utilisation selon les revendications 1 à 7, pour traiter l'anxiété.

10. Agoniste du récepteur d'adiponectine pour une utilisation selon les revendications 1 à 7, pour traiter un trouble neuroinflammatoire.

11. Agoniste du récepteur d'adiponectine pour une utilisation selon la revendication 10, dans lequel le trouble neuroinflammatoire est une maladie neurodégénérative avec neuroinflammation.

12. Agoniste du récepteur d'adiponectine pour une utilisation selon la revendication 11, dans lequel la maladie neurodégénérative est choisie dans le groupe constitué par la maladie d'Alzheimer, la maladie de Parkinson, la sclérose en plaques, la sclérose latérale amyotrophique, la démence du lobe fronto-temporal et la maladie de Nasu-Hakola.

13. Agoniste du récepteur d'adiponectine pour une utilisation selon les revendications 1 à 12, dans lequel le composé doit être administré par voie orale.
